# EUROPEAN PATENT APPLICATION

(11) **EP 1 941 905 A1**
(43) Date of publication of application: **09.07.2008**
(21) Application number: 08005672.4
(22) Date of filing: 26.03.1999
(51) Int. Cl.: A61K 39/395, A61K 38/17, C07K 14/705, C07K 16/32

(54) **APO-2 Ligand-anti-her-2 antibody synergism**

(30) Priority: 27.03.1998 US 79683 P
(62) Divisional of application: 99915054.3
(71) Applicant: GENENTECH, INC., South San Francisco, CA 94080-4990 (US)
(72) Inventor: The designation of the inventor has not yet been filed
(74) Representative: Woolley, Lindsey Claire

(57) **Abstract**

Methods of using synergistically effective amounts of Apo-2 ligand and anti-Her-2 antibodies to enhance cell death via apoptosis are provided.

## Description

### Field of the Invention

This invention relates generally to methods of inducing apoptosis in mammalian cells. In particular, it pertains to the use of Apo-2 ligand and anti-Her-2 antibody to synergistically induce apoptosis in mammalian cells.

### Background of the Invention

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, e.g., Barr et al., Bio/Technology, 12:487-493 (1994)]. Apoptotic cell death naturally occurs in many physiological processes, including embryonic development and clonal selection in the immune system [Itoh et al., Cell, 66:233-243 (1991)]. Decreased levels of apoptotic cell death, however, have been associated with a variety of pathological conditions, including cancer, lupus, and herpes virus infection [Thompson, Science, 267:1456-1462 (1995)].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, Science, 267:1445-1449 (1995); Sachs et al., Blood, 82:15 (1993)]. For instance, they can be triggered by hormonal stimuli, such as glucocorticoid hormones for immature thymocytes, as well as withdrawal of certain growth factors [Watanabe-Fukunaga et al., Nature, 356:314-317 (1992)]. Also, some identified oncogenes such as *myc, rel,* and *E1A,* and tumor suppressors, like *p53,* have been reported to have a role in inducing apoptosis. Certain chemotherapy drugs and some forms of radiation have likewise been observed to have apoptosis-inducing activity [Thompson, supra].

Various molecules, such as tumor necrosis factor- α ("TNF-α"), tumor necrosis factor-β ("TNF-β " or "lymphotoxin"), CD30 ligand, CD27 ligand, CD40 ligand, OX-40 ligand, 4-1BB ligand, and Apo-1 ligand (also referred to as Fas ligand or CD95 ligand) have been identified as members of the tumor necrosis factor ("TNF") family of cytokines [See, e.g., Gruss and Dower, Blood, 85:3378-3404 (1995)]. Among these molecules, TNF-α, TNF-β, CD30 ligand, 4-1BB ligand, and Apo-1 ligand have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)].

Recently, additional molecules believed to be members of the TNF cytokine family were identified and reported to be involved in apoptosis. For instance, in Pitti et al., J. Biol. Chem., 271:12687-12690 (1996), a molecule referred to as Apo-2 Ligand is described. See also, WO 97/25428 published July 17, 1997. The full length human Apo-2 ligand is reported to be a 281 amino acid polypeptide that induces apoptosis in various mammalian cells. Other investigators have described related polypeptides referred to as TRAIL [Wiley et al., Immunity, 3:673-682 (1995); WO 97/01633 published January 16, 1997] and AGP-1 [WO 97/46686 published December 11, 1997)].

Several receptors for Apo-2 ligand have been described. These receptors include Apo-2 (also referred to as DR5) [Sheridan et al., Science, 277:818-821 (1997); Pan et al., Science, 277:815-818 (1997)], DR4 [Pan et al., Science, 276:111-113 (1997)], DcRl (also referred to as TRID) [Sheridan et al., Science, 277:818-821 (1997); Pan et al., Science, 277:815-818 (1997)] and DcR2 (also referred to as TRAIL-4) [Marsters et al., Current Biology, 7:1003-1006 (1997); Degli-Esposti et al., Immunity, 7:813-820 (1997)].

Molecules targeting a number of the growth factor receptor protein kinases have also been reported to induce apoptosis. The receptor protein tyrosine kinases, which fall into a number of subfamilies, are believed to have the primary function of directing cellular growth via ligand-stimulated tyrosine phosphorylation of intracellular substrates. The class I subfamily of growth factor receptor protein tyrosine kinases includes the 170 kDa epidermal growth factor receptor (EGFR) encoded by the *erb*B1 gene. *erb*B1 has been causally implicated in human malignancy. In particular, increased expression of this gene has been observed in carcinomas of the breast, bladder, lung, head, neck and stomach. Monoclonal antibodies directed against the EGFR have been evaluated as therapeutic agents in the treatment of such malignancies. For example, Wu et al., J. Clin. Invest., 95:1897-1905 (1995) recently reported that the anti-EGFR monoclonal antibody (mAb) 225 (which competitively inhibits EGF binding and blocks activation of this receptor) could induce the human colorectal carcinoma cell line DiFi (which expresses high levels of EGFR) to undergo G, cell cycle arrest and apoptosis. See Baselga et al., Pharmac. Ther., 64:127-154 (1994); Masui et al., Cancer research, 44:1002-1007 (1984).

The second member of the class I subfamily, p185*^{neu}*, was originally identified as the product of the transforming gene from neuroblastomas of chemically treated rats. The activated form of the *neu* protooncogene results from a point mutation (valine to glutamic acid) in the transmembrane region of the encoded protein. Amplification of the human homolog of *neu* (called Her-2 or *erb*B2) is observed in breast and ovarian cancers and generally correlates with a poor prognosis [Slamon et al., Science, 235:177-182 (1987); Slamon et al., Science, 244:707-712 (1989)]. Accordingly, Slamon et al. in U.S. Pat. No. 4,968,603 describe various diagnostic assays for determining Her-2 gene amplification or expression in tumor cells. To date, no point mutation analogous to that in the *neu* protooncogene has been reported for human tumors. Overexpression (frequently but not uniformly due to amplification) of Her-2 has also been observed in other carcinomas including carcinomas of the stomach, endometrium, salivary gland, lung, kidney, colon, thyroid, pancreas and bladder. See, among others, King et al., Science, 229:974 (1985); Yokota et al., Lancet, 1:765-767 (1986); Fukushigi et al., Mol. Cell. Biol., 6:955-958 (1986); Geurin et al., Oncogene Research, 3:21-31 (1988); Cohen et al., Oncogene, 4:81-88 (1989); Yonemura et al., Cancer Research, 51:1034 (1991); Borst et al., Gynecol. Oncol., 38:364 (1990); Weiner et al., Cancer Research, 50:421-425 (1990); Kern et al., Cancer Research, 50:5184 (1990); Park et al., Cancer Research, 49:6605 (1989); Zhau et al., Mol. Carcinog., 3:354-357 (1990); Aasland et al., Br. J. Cancer, 57:358-363 (1988); Williams et al., Pathobiology, 59:46-52 (1991); and McCann et al., Cancer, 65:88-92 (1990).

Certain antibodies directed against the rat *neu* and human Her-2 protein products have been described. Drebin et al., Cell, 41:695-706 (1985) refer to an IgG2a monoclonal antibody which is directed against the rat *neu* gene product. This antibody called 7.16.4 causes down-modulation of cell surface p185 expression on B104-1-1 cells (NIH-3T3 cells transfected with the *neu* protooncogene) and inhibits colony formation of these cells. In Drebin et al., Proc. Natl. Acad. Sci., 83:9129-9133 (1986), the 7.16.4 antibody was shown to inhibit the tumorigenic growth of *neu*-transformed NIH-3T3 cells as well as rat neuroblastoma cells (from which the *neu* oncogene was initially isolated) implanted into nude mice. Drebin et al., Oncogene, 2:387-394 (1988) discuss the production of a panel of antibodies against the rat *neu* gene product. All of the antibodies were found to exert a cytostatic effect on the growth of *neu*-transformed cells suspended in soft agar. Antibodies of the IgM, IgG2a and IgG2b isotypes were able to mediate *in vitro* lysis of *neu-*transformed cells in the presence of complement, whereas none of the antibodies were able to mediate relatively high levels of antibody-dependent cellular cytotoxicity (ADCC) of the *neu*-transformed cells. Drebin et al., Oncogene, 2:273-277 (1988) report that mixtures of antibodies reactive with two distinct regions on the p185 molecule result in synergistic anti-tumor effects on *neu*-transformed NIH-3T3 cells implanted into nude mice. Biological effects of anti-*neu* antibodies are reviewed in Myers et al., Meth. Enzym., 198:277-290 (1991). See also WO94/22478 published October 13, 1994.

Hudziak et al., Mol. Cell. Biol., 9(3):1165-1172 (1989) describe the generation of a panel of anti-Her-2 antibodies which were characterized using the human breast tumor cell line SKBR3. Relative cell proliferation of the SKBR3 cells following exposure to the antibodies was determined by crystal violet staining of the monolayers after 72 hours. Using this assay, maximum inhibition was obtained with the antibody called 4D5 which inhibited cellular proliferation by 56%. Other antibodies in the panel, including 7C2 and 7F3, reduced cellular proliferation to a lesser extent in this assay. Hudziak et al. conclude that the effect of the 4DS antibody on SKBR3 cells was cytostatic rather than cytotoxic, since SKBR3 cells resumed growth at a nearly normal rate following removal of the antibody from the medium. The antibody 4D5 was further found to sensitize p185^{*erb*^{B2}}-overexpressing breast tumor cell lines to the cytotoxic effects of TNF-α. See also WO89/06692 published July 27, 1989. The anti-Her-2 antibodies discussed in Hudziak et al. are further characterized in Fendly et al., Cancer Research, 50:1550-1558 (1990); Kotts et al., In Vitro, 26(3): 59A (1990); Sarup et al., Growth Regulation, 1:72-82 (1991); Shepard et al., J. Clin. Immunol., 11(3):117-127 (1991); Kumar et al., Mol. Cell. Biol., 11(2):979-986 (1991); Lewis et al., Cancer Immunol. Immunotherap., 37:255-263 (1993); Pietras et al., Oncogene, 9:1829-1838 (1994); Vitetta et al., Cancer Research, 54:5301-5309 (1994); Sliwkowski et al., J. Biol. Chem., 692(20):14661-14665 (1994); Scott et al., J. Biol. Chem., 266:14300-5 (1991); and D'souza et al., Proc. Natl. Acad. Sci., 91:7202-7206 (1994).

Certain anti-Her-2 antibodies can induce death of a Her-2 overexpressing cell (e.g. a BT474, SKBR3, SKOV3 or Calu 3 cell) via apoptosis. Ghetie et al., Proc. Natl. Acad. Sci., 94:7509-7514 (1997) discuss the production of an anti-Her-2 antibody which, when homodimerized, induces apoptosis in tumor cells. Further, Kita et al., Biochem. Biophys. Research Commun. discuss the generation of an anti-Her-2 antibody which induced cell morphology changes and apoptosis in cells transfected with the Her-2 gene. In contrast to the apoptotic anti-EGFR antibody described in Wu et al., J. Clin. Investigation, 95:1897-1905 (1995), those anti-Her-2 antibodies are not thought to induce apoptosis by disruption of an autocrine loop.

Other antibodies specific for Her-2 have been described in the art. Tagliabue et al., Int. J. Cancer, 47:933-937 (1991) describe two antibodies which were selected for their reactivity on the lung adenocarcinoma cell line (Calu-3) which overexpresses Her-2. One of the antibodies, called MGR3, was found to internalize, induce phosphorylation of Her-2, and inhibit tumor cell growth *in vitro.*

McKenzie et al., Oncogene, 4:543-548 (1989) generated a panel of anti-Her-2 antibodies, including the antibody designated TA1. This TA1 antibody was found to induce accelerated endocytosis of Her-2 [see Maier et al., Cancer Research, 51:5361-5369 (1991)]. Bacus et al., Mol. Carcinogenesis, 3:350-362 (1990) reported that the TA1 antibody induced maturation of the breast cancer cell lines AU-565 (which overexpresses the Her-2 gene) and MCF-7. Inhibition of growth and acquisition of a mature phenotype in these cells was found to be associated with reduced levels of Her-2 receptor at the cell surface and transient increased levels in the cytoplasm.

Stancovski et al., Proc. Natl. Acad. Sci., 88:8691-8695 (1991) generated a panel of anti-Her-2 antibodies, injected them i.p. into nude mice and evaluated their effect on tumor growth of murine fibroblasts transformed by overexpression of the Her-2 gene. Various levels of tumor inhibition were detected for four of the antibodies, but one of the antibodies (N28) consistently stimulated tumor growth. Monoclonal antibody N28 induced significant phosphorylation of the Her-2 receptor, whereas the other four antibodies generally displayed low or no phosphorylation-inducing activity. The effect of the anti-Her-2 antibodies on proliferation of SKBR3 cells was also assessed. In this SKBR3 cell proliferation assay, two of the antibodies (N12 and N29) caused a reduction in cell proliferation relative to control. The ability of the various antibodies to induce cell lysis *in vitro* via complement-dependent cytotoxicity (CDC) and antibody-mediated cell-dependent cytotoxicity (ADCC) was assessed, with the authors of this paper concluding that the inhibitory function of the antibodies was not attributed significantly to CDC or ADCC.

Bacus et al., Cancer Research, 52:2580-2589 (1992) further characterized the antibodies described in Bacus et al. (1990) and Stancovski et al. cited above. Extending the i.p. studies of Stancovski et al., the effect of the antibodies after i.v. injection into nude mice harboring mouse fibroblasts overexpressing human Her-2 was assessed. As observed in their earlier work, N28 accelerated tumor growth whereas N12 and N29 significantly inhibited growth of the Her-2-expressing cells. Partial tumor inhibition was also observed with the N24 antibody. Bacus et al. also tested the ability of the antibodies to promote a mature phenotype in the human breast cancer cell lines AU-565 and MDA-MB453 (which overexpress Her-2) as well as MCF-7 (containing low levels of the receptor). Bacus et al. saw a correlation between tumor inhibition *in vivo* and cellular differentiation; the tumor-stimulatory antibody N28 had no effect on differentiation, and the tumor inhibitory action of the N12, N29 and N24 antibodies correlated with the extent of differentiation they induced.

Xu et al., Int. J. Cancer, 53:401-408 (1993) evaluated a panel of anti-Her-2 antibodies for their epitope binding specificities, as well as their ability to inhibit anchorage-independent and anchorage-dependent growth of SKBR3 cells (by individual antibodies and in combinations), modulate cell-surface Her-2, and inhibit ligand stimulated anchorage-independent growth. See also WO94/00136 published Jan 6, 1994 and Kasprzyk et al., Cancer Research, 52:2771-2776 (1992) concerning anti-Her-2 antibody combinations. Other anti-Her-2 antibodies are discussed in Hancock et al., Cancer Research, 51:4575-4580 (1991); Shawver et al., Cancer Research, 54:1367-1373 (1994); Arteaga et al., Cancer Research, 54:3758-3765 (1994); and Harwerth et al., J. Biol. Chem., 267:15160-15167 (1992).

A further gene related to *Her-2,* called *erb*B3 or HER3, has also been described. See, e.g., U.S. Pat. Nos. 5,183,884 and 5,480,968. ErbB3 is unique among the ErbB receptor family in that it possesses little or no intrinsic tyrosine kinase activity. However, when ErbB3 is co-expressed with Her-2, an active signaling complex is formed and antibodies directed against Her-2 are capable of disrupting this complex [Sliwkowski et al., J. Biol.. Chem., 269(20): 14661-14665 (1994)]. Additionally, the affinity of ErbB3 for heregulin (HRG) is increased to a higher affinity state when co-expressed with Her-2. See also, Levi et al., J. Neuroscience, 15: 1329-1340 (1995); Morrissey et al., Proc. Natl. Acad. Sci., 92: 1431-1435 (1995); and Lewis et al., Cancer Research, 56:1457-1465 (1996) with respect to the Her-2-ErbB3 protein complex.

The class I subfamily of growth factor receptor protein tyrosine kinases has been further extended to include the HER4/p180^{*erb*B4} receptor. See EP Patent Application No. 599,274; Plowman et al., Proc. Natl. Acad. Sci., 90:1746-1750 (1993); and Plowman et al., Nature, 366:473-475 (1993). Plowman et al. found that increased HER4 expression correlated with certain carcinomas of epithelial origin, including breast adenocarcinomas. This receptor, like ErbB3, forms an active signalling complex with Her-2 [Carraway and Cantley, Cell, 78:5-8 (1994)].

### SUMMARY OF THE INVENTION

Applicants have surprisingly found that Apo-2 ligand and anti-Her-2 antibody can act synergistically to induce apoptosis in mammalian cells, particularly in mammalian cancer cells which overexpress Her-2.

The invention provides various methods for the use of Apo-2 ligand and anti-Her-2 antibody to induce apoptosis in mammalian cells. For example, the invention provides a method for inducing apoptosis comprising exposing a mammalian cell, such as a cancer cell which overexpresses Her-2, to Apo-2 ligand and anti-Her-2 antibody in an amount effective to synergistically induce apoptosis. The cell may be in cell culture or in a mammal, *e.g.* a mammal suffering from cancer. Thus, the invention includes a method for treating a mammal suffering from a condition characterized by overexpression of the Her-2 receptor, comprising administering an effective amount of Apo-2 ligand and anti-Her-2 antibody, as disclosed herein. According to any of the methods, one or more anti-Her-2 antibodies may be used. For instance, a first anti-Her-2 antibody such as the 7C2 antibody and a second anti-Her-2 antibody (different from the first antibody such as an antibody which binds to a different Her-2 epitope) may be employed. Preferably, at least one of the anti-Her-2 antibodies is an apoptosis-inducing antibody. Optionally, the methods may employ an agonistic anti-Apo-2 ligand receptor antibody which mimics the apoptotic activity of Apo-2 ligand.

The invention also provides compositions which comprise Apo-2 ligand and/or anti-Her-2 antibody(s). Optionally, the compositions of the invention will include pharmaceutically acceptable carriers or diluents. Preferably, the compositions will include Apo-2 ligand and/or anti-Her-2 antibody in an amount which is effective to synergistically induce apoptosis in mammalian cells.

The invention also provides articles of manufacture and kits which include Apo-2 ligand and/or anti-Her-2 antibody(s).

### Brief Description of the Drawings

Figure 1A shows a bar diagram illustrating the enhanced apoptotic activity (as determined by annexin V binding and uptake of PI) of Apo-2L and 7C2 antibody on BT474 and MCF7/HER2 breast tumor cells.
Figure 1B shows a bar diagram illustrating the enhanced apoptotic activity (as determined by annexin V binding and uptake of PI) of Apo-2L and 7C2 antibody on SKBR3 breast tumor cells.
Figure 2A shows a bar graph showing the decrease in SKBR3 viable cell number and the increased number of dead cells (as measured by trypan blue dye uptake) after treatment with Apo-2L and 7C2 antibody on SKBR3 breast tumor cells after 3 days.
Figure 2B shows a bar graph showing the decrease in SKBR3 viable cell number and the increased number of dead cells (as measured by trypan blue dye uptake) after treatment with Apo-2L and 7C2 antibody on SKBR3 breast tumor cells after 6 days.
Figure 3 shows a bar diagram illustrating the changes in BT474 breast tumor cell number (viable and dead cell number determined by trypan blue dye uptake) after treatment with Apo-2L and 7C2 antibody.

### Detailed Description of the Invention

### I. Definitions

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, and more specifically by binding of annexin V, fragmentation of DNA, cell shrinkage, dilation of endoplasmatic reticulum, cell fragmentation, and/or formation of membrane vesicles (called apoptotic bodies).

As used herein, the term "synergy" or "synergism" or "synergistically" refers to the interaction of two or more agents so that their combined effect is greater than the sum of their individual effects.

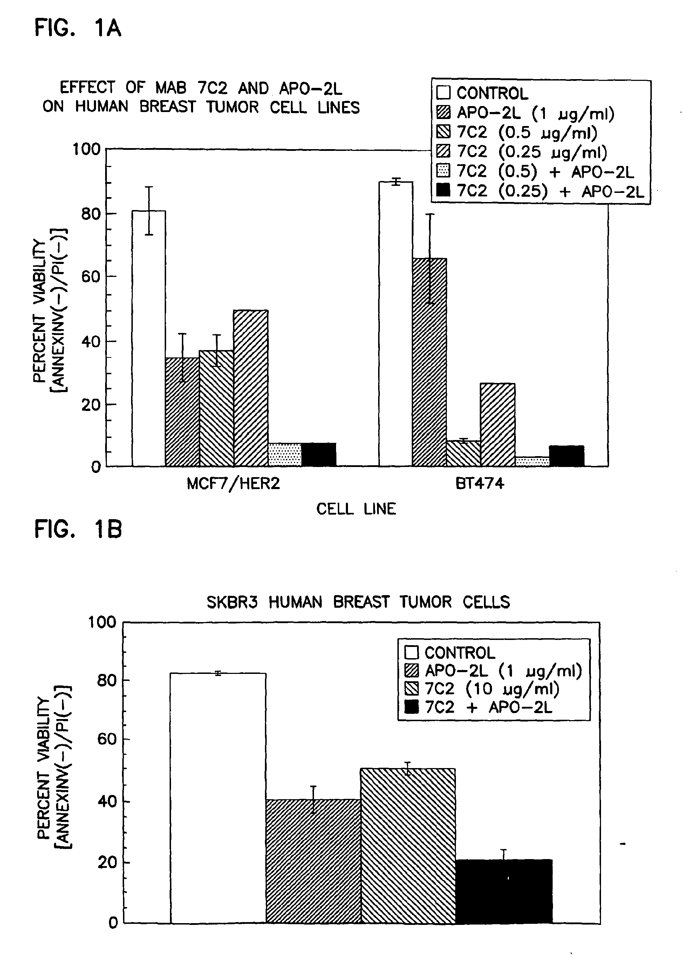

The terms "Apo-2 ligand" and "Apo-2L" are used herein to refer to a polypeptide which includes amino acid residues 114-281, inclusive, residues 91-281, inclusive, residues 92-281, inclusive, residues 41-281, inclusive, residues 15-281, inclusive, or residues 1-281, inclusive, of the amino acid sequence shown in Figure 1A of Pitti et al., J. Biol. Chem., 271:12687-12690 (1996), as well as biologically active deletional, insertional, or substitutional variants of the above sequences. In one embodiment, the polypeptide sequence has at least residues 114-281. Optionally, the polypeptide sequence has at least residues 91-281 or residues 92-281. In another preferred embodiment, the biologically active variants have at least about 80% sequence identity, more preferably at least about 90% sequence identity, and even more preferably, at least about 95% sequence identity with any one of the above sequences. The definition encompasses Apo-2 ligand isolated from an Apo-2 ligand source, such as from human tissue types, or from another source, or prepared by recombinant or synthetic methods. The term Apo-2 ligand also refers to the polypeptides described in WO 97/25428, supra.

Unless indicated otherwise, the term "Her-2" when used herein refers to human Her-2 protein and human Her-2 gene. The human Her-2 gene and Her-2 protein are described in Semba et al., Proc. Natl. Acad. Sci., 82:6497-6501 (1985) and Yamamoto et al., Nature, 319:230-234 (1986) (Genebank accession number X03363), for example. Her-2 comprises four domains (Domains 1-4). "Domain 1" is at the amino terminus of the extracellular domain of Her-2. See Plowman et al., Proc. Natl. Acad. Sci., 90:1746-1750 (1993).

The "epitope 7C2/7F3" is the region at the N terminus of the extracellular domain of Her-2 to which the 7C2 and/or 7F3 antibodies (each deposited with the ATCC, see below) bind. To screen for antibodies which bind to the 7C2/7F3 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to establish whether the antibody binds to the 7C2/7F3 epitope on Her-2 (*i.e.* any one or more of residues in the region from about residue 22 to about residue 53 of Her-2).

The "epitope 4D5" is the region in the extracellular domain of Her-2 to which the antibody 4D5 (ATCC CRL 10463) binds. This epitope is close to the transmembrane region of Her-2. To screen for antibodies which bind to the 4D5 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to assess whether the antibody binds to the 4D5 epitope of Her-2 (i.e. any one or more residues in the region from about residue 529, e.g, about residue 561 to about residue 625, inclusive).

A cell which "overexpresses" Her-2 has significantly higher than normal Her-2 levels compared to a noncancerous cell of the same tissue type. Typically, the cell is a cancer cell, *e.g.* a breast, ovarian, stomach, endometrial, salivary gland, lung, kidney, colon, thyroid, pancreatic or bladder cell. The cell may also be a cell line such as SKBR3, BT474, Calu 3, MDA-MB-453, MDA-MB-361 or SKOV3.

"Heregulin" (HRG) when used herein refers to a polypeptide which activates the Her-2-ErbB3 and Her-2-ErbB4 protein complexes (i.e. induces phosphorylation of tyrosine residues in the complex upon binding thereto). Various heregulin polypeptides encompassed by this term are disclosed in Holmes et al., Science, 256:1205-1210 (1992); WO 92/20798; Wen et al., Mol. Cell. Biol., 14(3):1909-1919 (1994); and Marchionni et al., Nature, 362:312-318 (1993), for example. The term includes biologically active fragments and/or variants of a naturally occurring HRG polypeptide, such as an EGF-like domain fragment thereof (*e.g.* HRGβ1₁₇₇₋₂₄₄).

The "Her-2-ErbB3 protein complex" and "Her-2-ErbB4 protein complex" are noncovalently associated oligomers of the Her-2 receptor and the ErbB3 receptor or ErbB4 receptor, respectively. The complexes form when a cell expressing both of these receptors is exposed to HRG and can be isolated by immunoprecipitation and analyzed by SDS-PAGE as described in Sliwkowski et al., J. Biol. Chem., 269(20):14661-14665 (1994).

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light- chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al., NIH Publ. No.91-3242, Vol. I, pages 647-669 (1991)). The constant domains are not involved directly in binding an antibody to an antigen, but exhibit various effector functions, such as participation of the antibody in antibody-dependent cellular toxicity.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of immunoglobulins are called α, Δ, ε, γ, and µ, respectively. The subunit structures and three-dimensional configurations of different classes of immunoglobulins are well known.

The term "antibody" is used in the broadest sense and specifically covers intact monoclonal antibodies, polyclonal antibodies, multispecific antibodies (e.g. bispecific antibodies) formed from at least two intact antibodies, and antibody fragments so long as they exhibit the desired biological activity.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 (1995)); single-chain antibody molecules; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)).

"Humanized" forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED^{™} antibody wherein the antigen-binding region of the antibody is derived from an antibody produced by immunizing macaque monkeys with the antigen of interest.

"Single-chain Fv" or "ScFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the ScFv to form the desired structure for antigen binding. For a review of ScFv see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

As used herein, the term "salvage receptor binding epitope" refers to an epitope of the Fc region of an IgG molecule (*e.g*., IgG₁, IgG₂, IgG₃, or IgG₄) that is responsible for increasing the *in vivo* serum half-life of the IgG molecule.

"Isolated," when used to describe the various proteins disclosed herein, means protein that has been identified and separated and/or recovered from a component of its natural environment. Contaminant components of its natural environment are materials that would typically interfere with diagnostic or therapeutic uses for the protein, and may include enzymes, hormones, and other proteinaceous or non-proteinaceous solutes. In preferred embodiments, the protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated protein includes protein *in situ* within recombinant cells, since at least one component of the protein natural environment will not be present. Ordinarily, however, isolated protein will be prepared by at least one purification step.

"Treatment" or "therapy" refer to both therapeutic treatment and prophylactic or preventative measures.

"Mammal" for purposes of treatment or therapy refers to any animal classified as a mammal, including humans, domestic and farm animals, and zoo, sports, or pet animals, such as dogs, horses, cats, cows, etc. Preferably, the mammal is human.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastrointestinal cancer, renal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, liver cancer, prostate cancer, vulval cancer, thyroid cancer, hepatic carcinoma and various types of head and neck cancer.

### II. Methods and Materials

Generally, the methods of the invention for inducing apoptosis in mammalian cells comprise exposing the cells to an effective amount of Apo-2 ligand and anti-Her-2 antibody. Preferably, the amount of Apo-2L and anti-Her-2 antibody employed will be amounts effective to synergistically induce apoptosis. This can be accomplished *in vivo* or *ex vivo* in accordance, for instance, with the methods described below and in the Example. It is contemplated that the present invention may be used to treat various conditions, including those characterized by overexpression and/or activation of the Her-2 receptor. Exemplary conditions or disorders to be treated with the Apo-2 ligand and anti-Her-2 antibody include benign or malignant cancer. Methods of determining levels of Her-2 expression prior to exposing cells to Apo-2 ligand and anti-Her-2 antibody are well known in the art. For example Slamon et al. in U.S. Pat. No. 4,968,603 describe various diagnostic assays for determining *Her-2* gene amplification or expression in tumor cells.

### A. MATERIALS

The Apo-2L which can be employed in the methods includes the Apo-2L polypeptides described in Pitti et al., supra, and WO 97/25428, supra. It is contemplated that various forms of Apo-2L may be used, such as the full length polypeptide as well as soluble forms of Apo-2L which comprise an extracellular domain (ECD) sequence. Examples of such soluble ECD sequences include polypeptides comprising amino acids 114-281, 91-281 or 92-281 of the Apo-2L sequence shown in Figure 1A of Pitti et al., J. Biol. Chem., 271:12687-12690 (1996). It is presently believed that the polypeptide comprising amino acids 92-281 is a naturally cleaved form of Apo-2L. Applicants have expressed human Apo-2L in CHO cells and found that the 92-281 polypeptide is the expressed form of Apo-2L. Modified forms of Apo-2L, such as the covalently modified forms described in WO 97/25428 are included. In particular, Apo-2L linked to a non-proteinaceous polymer such as polyethylene glycol is included for use in the present methods. The Apo-2L polypeptide can be made according to any of the methods described in WO 97/25428.

It is contemplated that a molecule which mimics the apoptotic activity of Apo-2L may alternatively be employed in the presently disclosed methods. Examples of such molecules include agonistic antibodies which can induce apoptosis in a like manner to Apo-2L. In particular, these agonist antibodies would comprise antibodies to one or more of the receptors for Apo-2L and which can stimulate apoptosis. Agonist antibodies directed to at least one of these receptors, called Apo-2, have been prepared using fusion techniques such as described below. One of the Apo-2 receptor agonist antibodies is referred to as 3F11.39.7 and has been deposited with ATCC as deposit no. HB-12456 on January 13, 1998. Agonist activity of the Apo-2L receptor antibodies can be determined using various methods for assaying for apoptotic activity. Many of these apoptosis assays are described in further detail herein.

The anti-Her-2 antibodies which can be employed in the methods include monoclonal antibodies 7C2 and 7F3. It is contemplated that one or more anti-Her-2 antibodies can be used. Optionally, at least one of the anti-Her-2 antibodies will be an apoptosis-inducing antibody. Preferably, the antibody which induces apoptosis is one which results in about 2 to 50 fold, preferably about 5 to 50 fold, and most preferably about 10 to 50 fold, induction of annexin binding relative to untreated cells. A second anti-Her-2 antibody used in combination with a first anti-Her-2 antibody may, for instance, be an antibody which inhibits cell growth but does not induce apoptosis. Optionally, the antibodies will bind to a region in the extracellular domain of Her-2, *e.g.* to an epitope in Domain 1 of Her-2. Preferably, the antibodies will bind to the Her-2 epitope bound by the 7C2 and/or 7F3 antibodies described herein. Antibodies of particular interest are those which, in addition to the above-described properties, bind the Her-2 receptor with an affinity of at least about 10 nM, more preferably at least about 1nM.

The selected antibody may be one like 7C2 which binds specifically to human Her-2 and does not significantly cross-react with other proteins such as those encoded by the *erb*B1, *erb*B3 and/or *erb*B4 genes. Sometimes, the antibody may not significantly cross-react with the rat *neu* protein, *e.g.,* as described in Schecter et al., Nature, 312:513 (1984) and Drebin et al., Nature, 312:545-548 (1984). In such embodiments, the extent of binding of the antibody to these proteins (e.g., cell surface binding to endogenous receptor) will be less than about 10% as determined by fluorescence activated cell sorting (FACS) analysis or radioimmunoprecipitation (RIA).

Optionally the antibody will be one which blocks HRG binding/activation of the Her-2/ErbB3 complex (e.g. 7F3 antibody). Alternatively, the antibody is one which does not significantly block activation of the Her-2/ErbB3 receptor complex by HRG (e.g. 7C2). Further, the antibody may be one like 7C2 which does not induce a large reduction in the percent of cells in S phase (e.g. one which only induces about 0-10% reduction in the percent of these cells relative to control).

In one embodiment, the selected second antibody will inhibit growth of SKBR3 cells in cell culture by about 50% to 100% and will optionally bind to the epitope on Her-2 to which 4D5 antibody binds.

The Her-2 antigen to be used for production of antibodies may be, e.g., a soluble form of the extracellular domain of Her-2; a peptide such as a Domain 1 peptide or a portion thereof (e.g. comprising the 7C2 or 7F3 epitope). Alternatively, cells expressing Her-2 at their cell surface; or a carcinoma cell line such as SKBR3 cells, see Stancovski et al., PNAS (USA), 88:8691-8695 (1991)) can be used to generate antibodies. Other forms of Her-2 useful for generating antibodies will be apparent to those skilled in the art.

To identify or select for antibodies which induce apoptosis, loss of membrane integrity as indicated by, e.g., PI, trypan blue or 7AAD uptake is assessed relative to control. The preferred assay is the "PI uptake assay using BT474 cells". According to this assay, BT474 cells (which can be obtained from the American Type Culture Collection (Manassas, VA)) are cultured in Dulbecco's Modified Eagle Medium (D-MEM):Ham's F-12 (50:50) supplemented with 10% heat-inactivated FBS (Hyclone) and 2 mM L-glutamine. (Thus, the assay is performed in the absence of complement and immune effector cells). The BT474 cells are seeded at a density of 10⁶ per dish in 60 x 15 mm dishes and allowed to attach 2-3 days. The medium is then removed and replaced with fresh medium alone or medium containing 10 µg/ml of the appropriate MAb. The cells are incubated for a 3 day time period. Following each treatment, monolayers are washed with PBS and detached by trypsinization. Cells are then centrifuged at 1200 rpm for 5 minutes at 4°C, the pellet resuspended in 1 ml ice cold Ca²⁺ binding buffer (10 mM Hepes, pH 7.4, 140 mM NaCl, 2.5 mM CaCl₂) and aliquoted into 35 mm strainer-capped 12 x 75 tubes (1 ml per tube) for removal of cell clumps. Tubes then receive PI (0.1 µg/ml). Samples may be analyzed using a FACSCAN^{™} flow cytometer and FACSCONVERT^{™} CellQuest software (Becton Dickinson). Those antibodies which induce statistically significant levels of apoptosis as determined by PI uptake can be selected.

In order to select for antibodies which induce apoptosis, one can perform an annexin binding assay using BT474 cells as described in the Example below. The BT474 cells are cultured and seeded in dishes as discussed in the preceding paragraph. The medium is then removed and replaced with fresh medium alone or medium containing 10 µg/ml of the MAb. Following a three day incubation period, monolayers are washed with PBS and detached by trypsinization. Cells are then centrifuged, resuspended in Ca²⁺ binding buffer and aliquoted into tubes as discussed above for the cell death assay. Tubes then receive labelled annexin (e.g. annexin V-FITC) (1 µg/ml). Samples may be analyzed using a FACSCAN^{™} flow cytometer and FACSCONVERT^{™} CellQuest software (Becton Dickinson). Those antibodies which induce statistically significant levels of annexin binding relative to control are selected as apoptosis-inducing antibodies.

In addition to the annexin binding assay discussed in the preceding paragraph, a DNA staining assay using BT474 cells may be utilized. In order to perform this assay, BT474 cells which have been treated with the antibody of interest as described in the preceding two paragraphs are incubated with 9 µg/ml HOECHST 33342^{™} for 2 hours at 37°C, then analyzed on an EPICS ELITE^{™} flow cytometer (Coulter Corporation) using MODFIT LT^{™} software (Verity Software House). Antibodies which induce a change in the percentage of apoptotic cells which is 2 fold or greater (and preferably 3 fold or greater) than untreated cells (up to 100% apoptotic cells) may be selected as apoptotic antibodies using this assay.

To identify or select for antibodies which bind to an epitope on Her-2 bound by an antibody of interest, a routine cross-blocking assay such as that described in Antibodies, A Laboratory Manual, Cold Spring Harbor Laboratory, Ed Harlow and David Lane (1988), can be performed. Alternatively, epitope mapping can be performed.

To identify anti-Her-2 antibodies which inhibit growth of SKBR3 cells in cell culture by 50-100%, the SKBR3 assay described in WO89/06692 can be performed. According to this assay, SKBR3 cells are grown in a 1:1 mixture of F12 and DMEM medium supplemented with 10% fetal bovine serum, glutamine and penicillin/streptomycin. The SKBR3 cells are plated at 20,000 cells in a 35mm cell culture dish (2 mls/35mm dish). 2.5 µg/ml of the anti-Her-2 antibody is added per dish. After six days, the number of cells, compared to untreated cells are counted using an electronic COULTER^{™} cell counter. Those antibodies which inhibit growth of the SKBR3 cells by 50-100% can be selected for combination with the apoptotic antibodies as desired. Alternative methodologies for evaluating the growth inhibition of cells such as SKBR3 are also known in the art, for example those which utilize crystal violet to stain cells. See e.g. Phillips et al., Cancer Immunol. Immunother. 37:255-263 (1993).

Various types of Her-2 antibodies may be used in the methods, and such types of antibodies are described generally below in subsections (i)-(vii).

### (i) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R' are different alkyl groups.

Animals are immunized against the antigen, immunogenic conjugates, or derivatives by combining, *e.g.*, 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (ii) Monoclonal antibodies

Monoclonal antibodies are obtained from a population of substantially homogeneous antibodies, *i.e.,* the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Manassas, Virginia USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs., 130:151-188 (1992).

In a further embodiment, antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Patent No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iii) Humanized and human antibodies

Methods for humanizing non-human antibodies are well known in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-S25 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g*., Jakobovits et al., Proc. Natl. Acad. Sci. USA, 90:2551 (1993); Jakobovits et al., Nature, 362:255-258 (1993); Bruggermann et al., Year in Immuno., 7:33 (1993). Human antibodies can also be derived from phage-display libraries (Hoogenboom et al., J. Mol. Biol., 227:381 (1991); Marks et al., J. Mol. Biol., 222:581-597 (1991)).

### (iv) Antibody fragments

Various techniques have been developed for the production of antibody fragments. Traditionally, these fragments were derived via proteolytic digestion of intact antibodies (see, *e.g*., Morimoto et al., Journal of Biochemical and Biophysical Methods, 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology, 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185.

### (v) Bispecific antibodies

Bispecific antibodies are antibodies that have binding specificities for at least two different epitopes. Exemplary bispecific antibodies may bind to two different epitopes of the Her-2 protein. For example, one arm may bind an epitope in Domain 1 of Her-2 such as the 7C2/7F3 epitope, the other may bind a different Her-2 epitope, e.g. the 4D5 epitope. Other such antibodies may combine a Her-2 binding site with binding site(s) for EGFR, ErbB3 and/or ErbB4. Alternatively, an anti-Her-2 arm may be combined with an arm which binds to a triggering molecule on a leukocyte such as a T-cell receptor molecule (e.g. CD2 or CD3), or Fc receptors for IgG (FcγR), such as FcγRI (CD64), FcγRII (CD32) and FcγRIII (CD16) so as to focus cellular defense mechanisms to the Her-2-expressing cell. Bispecific antibodies may also be used to localize cytotoxic agents to cells which express Her-2. These antibodies possess an Her-2-binding arm and an arm which binds the cytotoxic agent (*e.g.* saporin, anti-interferon-α, vinca alkaloid, ricin A chain, methotrexate or radioactive isotope hapten). Bispecific antibodies can be prepared as full length antibodies or antibody fragments (e.g. F(ab')₂ bispecific antibodies).

Optionally, the bispecific antibodies may include antibodies which combine a Her-2 binding site with binding site(s) for an Apo-2L receptor. Such receptors would include the Apo-2 receptor and DR4 receptor (which are described in the Background). For example, the bispecific antibody may have one arm which binds a Her-2 epitope and another arm which binds a receptor for Apo-2 ligand.

Methods for making bispecific antibodies are known in the art. Traditional production of full length bispecific antibodies is based on the coexpression of two immunoglobulin heavy chain-light chain pairs, where the two chains have different specificities (Milstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986). According to another approach described in W096/27011, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from *E*. *coli* and subjected to directed chemical coupling *in vitro* to form the bispecific antibody. The bispecific antibody thus formed was able to bind to cells overexpressing the Her-2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol., 147: 60 (1991).

### (vi) Effector function engineering

It may be desirable to modify the antibody of the invention with respect to effector function, so as to enhance the effectiveness of the antibody in treating cancer, for example. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B., J. Immunol., 148:2918-2922 (1992). Homodimeric antibodies with enhanced anti-tumor activity may also be prepared using heterobifunctional crosslinkers as described in Wolff et al., Cancer Research, 53:2560-2565 (1993). Alternatively, an antibody can be engineered which has dual Fc regions and may thereby have enhanced complement lysis and ADCC capabilities. See Stevenson et al., Anti-Cancer Drug Design, 3:219-230 (1989).

### (vii) Antibody-salvage receptor binding epitope fusions.

In certain embodiments of the invention, it may be desirable to use an antibody fragment, rather than an intact antibody, to increase tumor penetration, for example. In this case, it may be desirable to modify the antibody fragment in order to increase its serum half life. This may be achieved, for example, by incorporation of a salvage receptor binding epitope into the antibody fragment (e.g. by mutation of the appropriate region in the antibody fragment or by incorporating the epitope into a peptide tag that is then fused to the antibody fragment at either end or in the middle, e.g., by DNA or peptide synthesis).

A systematic method for preparing such an antibody variant having an increased *in vivo* half-life comprises several steps. The first involves identifying the sequence and conformation of a salvage receptor binding epitope of an Fc region of an IgG molecule. Once this epitope is identified, the sequence of the antibody of interest is modified to include the sequence and conformation of the identified binding epitope. After the sequence is mutated, the antibody variant is tested to see if it has a longer *in vivo* half-life than that of the original antibody. If the antibody variant does not have a longer *in vivo* half-life upon testing, its sequence is further altered to include the sequence and conformation of the identified binding epitope. The altered antibody is tested for longer *in vivo* half-life, and this process is continued until a molecule is obtained that exhibits a longer *in vivo* half-life.

The salvage receptor binding epitope being thus incorporated into the antibody of interest is any suitable such epitope as defined above, and its nature will depend, e.g., on the type of antibody being modified. The transfer is made such that the antibody of interest still possesses the biological activities described herein.

The epitope preferably constitutes a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (e.g., of an IgG) and transferred to the CH1, CH3, or V_{H} region, or more than one such region, of the antibody. Alternatively, the epitope is taken from the CH2 domain of the Fc region and transferred to the C_{L} region or V_{L} region, or both, of the antibody fragment.

### B. FORMULATIONS

The Apo-2 ligand and anti-Her-2 antibody are preferably administered in a carrier. Both can be administered in a single carrier, or alternatively, can be included in separate carriers. Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the carrier to render the formulation isotonic. Examples of the carrier include saline, Ringer's solution and dextrose solution. The pH of the solution is preferably from about 5 to about 8, and more preferably from about 7.4 to about 7.8. It will be apparent to those persons skilled in the art that certain carriers may be more preferable depending upon, for instance, the route of administration and concentration of agent being administered. The carrier may be in the form of a lyophilized formulation or aqueous solution.

Acceptable carriers, excipients, or stabilizers are preferably nontoxic to cells and/or recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG).

The formulation may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The Apo-2L and/or anti-Her-2 antibody may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

The formulations to be used for *in vivo* administration should be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

### C. MODES OF ADMINISTRATION

The Apo-2L and Her-2 antibody can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering Apo-2 ligand and Her-2 antibody may be determined empirically, and making such determinations is within the skill in the art. It is presently believed that an effective dosage or amount of Apo-2 ligand used alone may range from about 1 µg/kg to about 100 mg/kg of body weight or more per day. Interspecies scaling of dosages can be performed in a manner known in the art, e.g., as disclosed in Mordenti et al., Pharmaceut. Res., 8:1351 (1991). Those skilled in the art will understand that the dosage of Apo-2 ligand that must be administered will vary depending on, for example, the mammal which will receive the Apo-2 ligand, the route of administration, and other drugs or therapies being administered to the mammal.

Depending on the type of cells and/or severity of the disease, about 1 µg/kg to 15 mg/kg (e.g. 0.1-20 mg/kg) of antibody is an initial candidate dosage for administration, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful.

For the prevention or treatment of disease, the appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

It is contemplated that yet additional therapies may be employed in the methods. The one or more other therapies may include but are not limited to, chemotherapy and/or radiation therapy, immunoadjuvants, cytokines, and other non-Her-2 antibody-based therapies. Examples include interleukins (e.g., IL-1, IL-2, IL-3, IL-6), leukemia inhibitory factor, interferons, TGF-beta, erythropoietin, thrombopoietin, and anti-VEGF antibody. Other agents known to induce apoptosis in mammalian cells may also be employed, and such agents include TNF-α, TNF-β (lymphotoxin-α), CD30 ligand, 4-1BB ligand, and Apo-1 ligand.

Chemotherapies contemplated by the invention include chemical substances or drugs which are known in the art and are commercially available, such as Adriamycin, Doxorubicin, 5-Fluorouracil, Cytosine arabinoside ("Ara-C"), Cyclophosphamide, Camptothecin, Leucovorin, Thiotepa, Busulfan, Cytoxin, Taxol, Toxotere, Methotrexate, Cisplatin. Melphalan, Vinblastine, Bleomycin, Etoposide, Ifosfamide, Mitomycin C, Mitoxantrone, Vincreistine, Vinorelbine, Carboplatin, Teniposide, Daunomycin. Carminomycin, Aminopterin, Dactinomycin, Mitomycins, Esperamicins (see U.S. Pat. No. 4,675,187), Melphalan and other related nitrogen mustards. Also included are agents that act to regulate or inhibit hormone action on tumors such as tamoxifen and onapristone.

Preparation and dosing schedules for such chemotherapy may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration with the Apo-2L and/or Her-2 antibody or may be given simultaneously therewith.

The chemotherapy is preferably administered in a carrier, such as those described above. The mode of administration of the chemotherapy may be the same as employed for the Apo-2 ligand or Her-2 antibody, or it may be administered via a different mode.

Radiation therapy can be administered according to protocols commonly employed in the art and known to the skilled artisan. Such therapy may include cesium, iridium, iodine, or cobalt radiation. The radiation therapy may be whole body irradiation, or may be directed locally to a specific site or tissue in or on the body. Typically, radiation therapy is administered in pulses over a period of time from about 1 to about 2 weeks. The radiation therapy may, however, be administered over longer periods of time. Optionally, the radiation therapy may be administered as a single dose or as multiple, sequential doses.

The Apo-2 ligand and anti-Her-2 antibody (and one or more other therapies) may be administered concurrently or sequentially. Following administration of Apo-2 ligand and Her-2 antibody, treated cells *in vitro* can be analyzed. Where there has been *in vivo* treatment, a treated mammal can be monitored in various ways well known to the skilled practitioner. For instance, tumor mass may be observed physically, by biopsy or by standard x-ray imaging techniques.

### III. Articles of Manufacture

In another embodiment of the invention, an article of manufacture containing materials useful for the treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agents in the composition are the Apo-2 ligand and anti-Her-2 antibodies. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

The following examples are offered by way of illustration and not by way of limitation. The disclosures of all citations in the specification are expressly incorporated herein by reference.

### EXAMPLE 1

**Cell lines**. The established human breast tumor cells BT474 (available from ATCC) and MCF7/HER-2 (HER-2 transfected MCF7 breast tumor cell line; SKBR3 and MCF7 cells available from ATCC) were grown in DMEM:Ham's F-12 (50:50) (Gibco, Grand Island, NY) supplemented with 10% heat-inactivated fetal bovine serum (FBS) (HyClone, Logan, UT), and L-glutamine (2 mM).

**Biochemical reagents**. Bicohemical reagents used for the apoptosis studies were: annexin V-FITC (BioWhittaker, Inc.), propidium iodide (PI, Molecular Probes, Inc.), and HOECHST 33342^{™} (Calbiochem).

Apo-2 ligand. His-tagged Apo-2L comprising amino acids 114-281 of Apo-2L (as shown in Figure 1A of Pitti et al., J. Biol. Chem., 271:12687-12690 (1996)) was prepared as described in WO 97/25428.

**Antibodies.** The anti-Her-2 IgG₁K murine monoclonal antibodies specific for the extracellular domain of Her-2, were produced as described in Fendly et al., Cancer Research, 50:1550-1558 (1990) and WO89/06692. Briefly, NIH 3T3/HER-2-3₄₀₀ cells (expressing approximately 1 x 10⁶ Her-2 molecules/cell) produced as described in Hudziak et al., Proc. Natl. Acad. Sci. (USA), 84:7159 (1987) were harvested with phosphate buffered saline (PBS) containing 25 mM EDTA and used to immunize BALB/c mice. The mice were given i.p. injections of 10⁷ cells in 0.5ml PBS on weeks, 0, 2, 5 and 7. The mice with antisera that immunoprecipitated ³²P-labeled Her-2 were given i.p. injections of a wheat germ agglutinin-Sepharose (WGA) purified Her-2 membrane extract on weeks 9 and 13. This was followed by an i.v. injection of 0.1 ml of the Her-2 preparation and the splenocytes were fused with mouse myeloma line X63-Ag8.653. Hybridoma supernatants were screened for Her-2-binding by ELISA and radioimmunoprecipitation. MOPC-21 (IgG1), (Cappell, Durham, NC), was used as an isotype-matched control.

The anti-Her-2 MAb used is designated 7C2. The isotype-matched control MAb 1766 is directed against the herpes simplex virus (HSV-1) glycoprotein D. **Flow cytometry experiments for measuring induction of apoptosis**. BT474 cells were seeded at a density of 10⁶ per dish in 60 x 15 mm dishes and allowed to attach 2-3 days. SKBR3 cells were seeded at a density of 5.0 x 10⁵ per dish in 60 x 15 mm dishes and allowed to attach 2-3 days The medium was then removed and replaced with fresh medium alone or medium containing Apo-2 ligand and the mAb designated 7C2. For most experiments, cells were incubated for a 3 day time period. MAb concentrations used in the experiments were 0.25, 0.5, 1 and 10 µg/ml. The Apo-2 ligand concentration used in the experiments was 1 µg/ml. Following each-treatment, supernatants were individually collected and kept on ice, monolayers were detached by trypsinization and pooled with the corresponding supernatant. Cells were then centrifuged at 1200 rpm for 5 minutes at 4°C, the pellet resuspended in 1 ml ice cold Ca²⁺ binding buffer (10 mM Hepes, pH 7.4, 140 mM Nad, 2.5 mM CaCl₂) and aliquoted into 35 mm strainer-capped 12 x 75 tubes (1 ml per tube) for removal of cell aggregates. Each tube then received annexin V-FITC (0.1 µg/ml) or PI (10 µg/ml) or annexin V-FITC plus PI or trypan blue. Samples were analyzed using a FACSCAN^{™} flow cytometer and FACSCONVERT^{™} CellQuest software (Becton Dickinson).

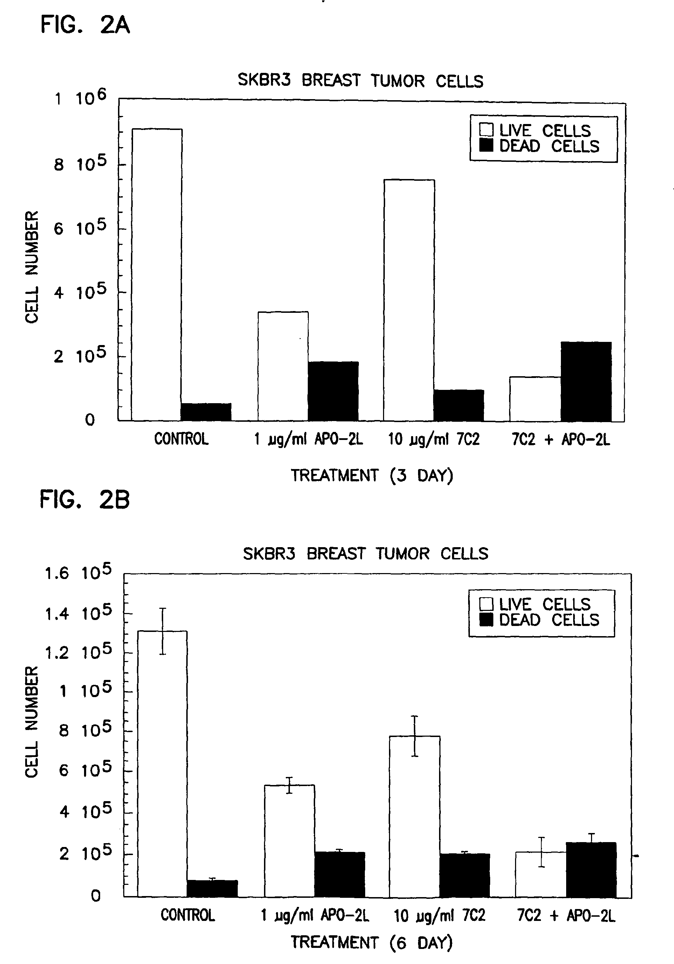

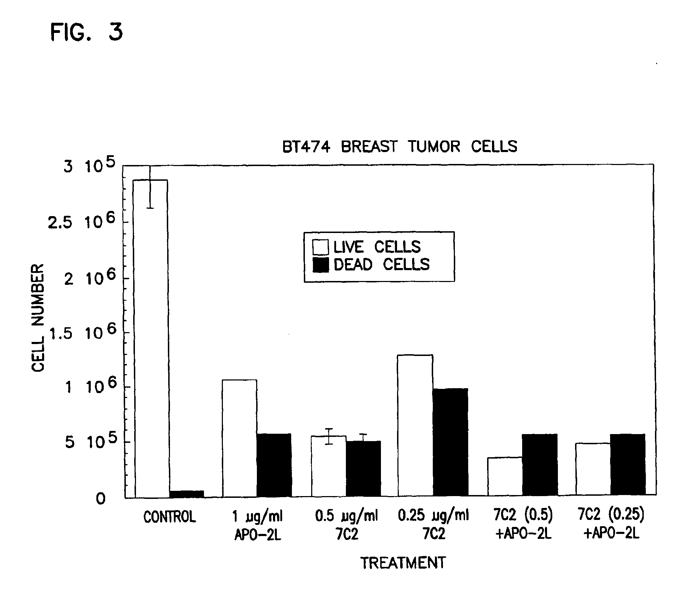

The results of the experiments are shown in Figures 1-3. Combinations of the Apo-2 ligand and the 7C2 anti-Her-2 MAb synergistically induced apoptosis in the cell lines which overexpress Her-2 as evidenced by annexin-V binding, PI uptake or trypan blue uptake.

### Deposit of Materials

The following materials have been deposited with the American Type Culture Collection, 10801 University Boulevard, Manassas, Virginia, USA (ATCC):

| **Antibody Designation** | **ATCC No.** | **Deposit Date** |
|---|---|---|
| 7C2 | ATCC HB-12215 | October 17, 1996 |
| 7F3 | ATCC HB-12216 | October 17, 1996 |
| 4D5 | ATCC CRL 10463 | May 24, 1990 |
| Apo-2L | ATCC CRL 12014 | January 3, 1996 |

These deposits were made under the provisions of the Budapest Treaty on the International Recognition of the Deposit of Microorganisms for the Purpose of Patent Procedure and the Regulations thereunder (Budapest Treaty). This assures maintenance of viable cultures for 30 years from the date of deposit. The deposits will be made available by ATCC under the terms of the Budapest Treaty, and subject to an agreement between Genentech, Inc. and ATCC, which assures (a) that access to the cultures will be available during pendency of the patent application to one determined by the Commissioner to be entitled thereto under 37 CFR §1.14 and 35 USC § 122, and (b) that all restrictions on the availability to the public of the cultures so deposited will be irrevocably removed upon the granting of the patent.

The assignee of the present application has agreed that if the cultures on deposit should die or be lost or destroyed when cultivated under suitable conditions, they will be promptly replaced on notification with a viable specimen of the same culture. Availability of the deposits is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the deposited materials. The deposit of material herein does not constitute an admission that the written description herein contained is inadequate to enable the practice of any aspect of the invention, including the best mode thereof, nor is it to be construed as limiting the scope of the claims to the specific illustration that it represents. Indeed, various modifications of the invention in addition to those shown and described herein will become apparent to those skilled in the art from the foregoing description and fall within the scope of the appended claims.

## Claims

1. A method of inducing apoptosis in cancer cells comprising exposing mammalian cancer cells expressing Her-2 receptor to a synergistically effective amount of Apo-2 ligand and anti-Her-2 antibody.

2. The method of claim 1 wherein the Apo-2 ligand is linked to a nonproteinaceous polymer.

3. The method of claim 2 wherein the polymer is polyethylene glycol.

4. The method of claim 1 wherein the anti-Her-2 antibody is an apoptotic antibody.

5. The method of claim 1 wherein the anti-Her-2 antibody binds to Domain 1 of Her-2.

6. The method of claim 1 wherein the anti-Her-2 antibody binds to epitope 7C2 on Her-2.

7. The method of claim 1 wherein the anti-Her-2 antibody is a monoclonal antibody.

8. The method of claim 1 wherein the anti-Her-2 antibody has nonhuman complementarity determining region (CDR) residues and human framework region (FR) residues.

9. The method of claim 1 wherein the anti-Her-2 antibody has complementarity determining regions (CDRs) of antibody 7C2.

10. The method of claim 1 wherein the anti-Her-2 antibody is a bispecific antibody.

11. The method of claim 10 wherein the bispecific antibody comprises an antibody having one arm which binds a Her-2 epitope and another arm which binds a receptor for Apo-2 ligand.

12. The method of claim 1 further comprising exposing the cells to one or more growth inhibitory agents.

13. The method of claim 1 further comprising exposing the cells to one or more chemotherapeutic agents.

14. The method of claim 1 further comprising exposing the cells to radiation.

15. The method of claim 1 wherein the cancer cells comprise breast cancer cells.

16. The method of claim 1 wherein the cancer cells comprise ovarian cancer cells.

17. The method of claim 1 wherein the cancer cells comprise lung cancer cells.

18. A method of inducing apoptosis in cancer cells comprising exposing mammalian cancer cells expressing Her-2 receptor to a synergistically effective amount of agonistic anti-Apo-2 ligand receptor antibody and anti-Her-2 antibody.

19. A method of treating cancer comprising exposing mammalian cancer cells expressing Her-2 receptor to a synergistically effective amount of Apo-2 ligand and anti-Her-2 antibody.

20. A composition comprising the Apo-2 ligand and anti-Her-2 antibody of claim 1 and a carrier.

21. The composition of claim 20 comprising an anti-Her-2 antibody which does not bind to Domain 1 of Her-2.

22. A kit comprising the Apo-2 ligand and anti-Her-2 antibody of claim 1 and instructions for using the Apo-2 ligand and the antibody to induce apoptosis in mammalian cancer cells.

23. An article of manufacture, comprising:
a container;
a label on the container;
an Apo-2 ligand contained within the container; and
an anti-Her-2 antibody contained within the container;
wherein the Apo-2 ligand and anti-Her-2 antibody are present in synergistically effective amounts and the label on the container indicates that combinations of the Apo-2 ligand and the anti-Her-2 antibody can be used for treating cancer cells which overexpress Her-2.
